# FASCICULE DE BREVET EUROPEEN

(11) **EP 0 808 187 B1**
(45) Date de publication et mention de la délivrance du brevet: **09.10.2002**
(21) Numéro de dépôt: 96941089.3
(22) Date de dépôt: 29.11.1996
(51) Int. Cl.: A61M 5/142, F04B 53/10

(54) **POMPE DE PERFUSION DE LIQUIDES MEDICAUX**
INFUSIONSPUMPE FÜR MEDIZINISCHE FLÜSSIGKEITEN
LIQUID DRUG INFUSION PUMP

(30) Priorité: 30.11.1995 FR 9514456
(43) Date de publication de la demande: 26.11.1997
(73) Titulaire: COMPAGNIE DE DEVELOPPEMENT AGUETTANT, F-69007 Lyon (FR)
(72) Inventeur: FREZZA, Pierre, F-69390 Charly (FR)
(74) Mandataire: Maureau, Philippe
(86) Numéro de dépôt international: FR9601906
(87) Numéro de publication internationale: WO97019716

(56) Documents cités:
- FR-A- 2 689 014

## Description

La présente invention a pour objet une pompe de perfusion de liquides médicaux.

Le document FR-A-2 689 014, au nom de la Demanderesse décrit une pompe de perfusion de liquides médicaux comprenant au moins un dispositif d'aspiration à cylindre et piston, d'un liquide dans un récipient et de refoulement de celui-ci dans une tubulure reliée au patient.

Cette pompe de perfusion comprend :
- une première pièce dans laquelle est ménagé au moins un cylindre de dosage qui, contenant un piston animé d'un mouvement alternatif, débouche dans une des faces de la pièce, perpendiculairement à cette face,
- une seconde pièce qui, prenant appui sur la face de la première pièce dans laquelle débouche chaque cylindre, comporte, en regard de chaque cylindre, une cavité de diamètre supérieur à celui du cylindre correspondant, et communiquant avec un récipient d'alimentation en liquide,
- une membrane étanche et déformable élastiquement serrée entre les deux pièces, et comportant un orifice en regard de chaque cylindre,
- un disque logé dans chaque cavité de la seconde pièce, dont la face tournée vers la première pièce est étagée et comprend une partie extérieure prenant appui, à travers la membrane, sur la face de la première pièce dans laquelle débouche le cylindre correspondant, juste autour de ce cylindre, et une partie centrale faisant saillie du côté de l'intérieur du cylindre, ce disque comportant des trous traversants régulièrement répartis et ménagés à proximité de sa périphérie,
- un ressort exerçant une pression sur le disque en direction de la première pièce, et
- associée à chaque cylindre, une lumière débouchant dans la même face de la première pièce que le cylindre considéré, cette lumière associée à une tubulure d'évacuation ménagée dans cette pièce, étant disposée à proximité du cylindre de dosage, en regard de la cavité de la seconde pièce, mais au-delà du disque que contient cette cavité, et étant recouverte par la membrane serrée entre les deux pièces.

Dans la pompe connue, il est nécessaire de prévoir un ressort exerçant une pression sur le disque en direction de la première pièce. La présence de ce ressort offre l'inconvénient du contact d'un matériau métallique avec le liquide médical transféré par la pompe. En outre, la présence de ce ressort au-dessus du disque impose que le guidage du disque soit réalisé au niveau de la périphérie de celui-ci dans la cavité ménagée dans la première pièce, ce qui conduit à une structure de disque étagée, et au ménagement de trous traversants dans le disque, à proximité de sa périphérie pour permettre le passage du liquide médical.

Un premier but de l'invention est de fournir une pompe de perfusion de liquides médicaux, dans laquelle le ressort prenant appui sur le disque en vue d'assurer l'étanchéité puisse être supprimé.

Un autre but de l'invention est de simplifier la structure du disque prenant appui contre la membrane.

A cet effet, la pompe de perfusion de liquides médicaux qu'elle concerne, du type comprenant :
- une première pièce dans laquelle est ménagé un cylindre de dosage qui, contenant un piston animé d'un mouvement alternatif, débouche dans une des faces de la pièce, perpendiculairement à cette face,
- une seconde pièce qui, prenant appui sur la face de la première pièce dans laquelle débouche le cylindre, comporte, en regard de celui-ci, une cavité de diamètre supérieur à celui du cylindre, et adaptée à communiquer, avec un récipient d'alimentation en liquide,
- une membrane étanche et déformable élastiquement serrée entre les deux pièces, et comportant un orifice en regard du cylindre,
- un disque logé dans la cavité de la seconde pièce, dont la face tournée vers la première pièce est adaptée à prendre appui sur la membrane,
- associée au cylindre, une lumière débouchant dans la même face de la première pièce que le cylindre, cette lumière associée à une tubulure d'évacuation ménagée dans cette pièce, étant disposée à proximité du cylindre, en regard de la même cavité de la seconde pièce, mais au-delà du cylindre de dosage, et étant recouverte par la membrane serrée entre les deux pièces,
est caractérisée en ce que la membrane comporte, sur sa face située du côté de l'amenée du liquide et dans la zone de la cavité ménagée dans la seconde pièce à l'extérieur du cylindre de dosage, une couronne élastique moulée avec la membrane et prenant appui dans le fond de la cavité ménagée dans la seconde pièce, le disque prenant appui sur la membrane étant d'un diamètre inférieur à celui de cette cavité et comportant des moyens de guidage central.

La présence de la couronne sur la membrane formant clapet remplace le ressort métallique connu dans le dispositif du document cité au préambule. La compression de cette couronne élastique crée l'étanchéité de la chambre à la pression d'utilisation de sortie.

Cette structure présente l'avantage de supprimer une pièce, en l'occurrence le ressort, et de n'avoir aucun matériau métallique en contact avec le liquide. En outre, la taille du disque peut être réduite, et celui-ci peut être guidé par son centre, dans la mesure où il n'y a plus de ressort agissant au niveau de ce centre.

Suivant une forme d'exécution de cette pompe, les moyens de guidage central du disque sont constitués par une tige perpendiculaire au disque et guidée en translation dans un conduit d'amenée du liquide.

Avantageusement dans ce cas, la tige de guidage du disque est de diamètre inférieur au diamètre du conduit d'amenée du liquide, et ce conduit comporte, au moins dans sa zone débouchant dans la pompe, des nervures axiales faisant saillie de sa paroi intérieure, délimitant, d'une part, une zone centrale de guidage de la tige du disque et, d'autre part, des canaux pour le passage du liquide.

Suivant une autre caractéristique de l'invention, les canaux ménagés dans la paroi du conduit d'amenée du liquide sont prolongés par des canaux radiaux ménagés dans le fond de la cavité de la seconde pièce et débouchant au-delà de la périphérie du disque.

De toute façon, l'invention sera bien comprise à l'aide de la description qui suit, en référence au dessin schématique annexé représentant, à titre d'exemple non limitatif, une forme d'exécution de cette pompe :

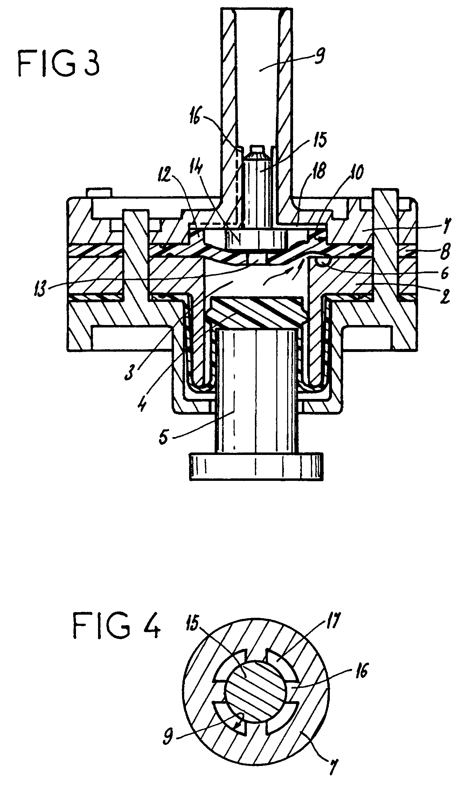

Figures 1 à 3 sont trois vues en coupe longitudinale de la pompe au cours de trois phases de fonctionnement ;
Figure 4 en est une vue en coupe transversale et à échelle agrandie selon la ligne IV-IV de figure 1.

La pompe de perfusion représentée au dessin comprend une première pièce 2 à l'intérieur de laquelle est ménagé un cylindre de dosage 3. A l'intérieur de ce cylindre de dosage est monté déplaçable axialement un piston 4 sous l'action d'un mécanisme d'entraînement schématisé et désigné par la référence 5. Dans la face supérieure de la pièce 2 débouche, à proximité du cylindre de dosage 3, une lumière 6 associée à une tubulure d'évacuation du liquide de perfusion, non représentée. La première pièce 2 est recouverte par une seconde pièce 7, montée avec interposition d'une membrane étanche 8. La seconde pièce 7 est prolongée en son centre par un conduit 9 d'amenée du liquide de perfusion. Dans la partie centrale de la seconde pièce 7 est ménagée une cavité 10 de diamètre supérieur au diamètre du cylindre de dosage 3, et dont la paroi périphérique est située au-delà de la lumière 6 d'évacuation du liquide. La membrane 8, qui est serrée entre les pièces 2 et 7, comporte, sur sa face située du côté de l'amenée du liquide et dans la zone de la cavité 10 située à l'extérieur du cylindre de dosage 3, une couronne élastique 12 moulée, prenant appui dans le fond de la cavité 10.

La membrane 8 présente également un orifice central 13. A l'intérieur de la cavité 10 est monté un disque 14 qui prend appui contre la face de la membrane tournée du côté de l'amenée du liquide. Comme montré au dessin, ce disque 14 est de diamètre inférieur à celui de la cavité 10. Ce disque 14 comprend, faisant saillie de son centre, une tige 15 engagée dans le conduit 9. Afin de pouvoir assurer le guidage de la tige 15 dans le conduit 9, celui-ci comporte, dans sa zone adjacente à la pompe, des nervures axiales 16, le guidage de la tige étant réalisé dans la partie centrale entre les nervures 16, et ces nervures délimitant entre elles des canaux 17 permettant le passage de liquide. Ces canaux 17 sont prolongés par des canaux radiaux 18 ménagés dans le fond de la cavité 10 de la seconde pièce 7, ces canaux débouchant au-delà de la périphérie du disque 14.

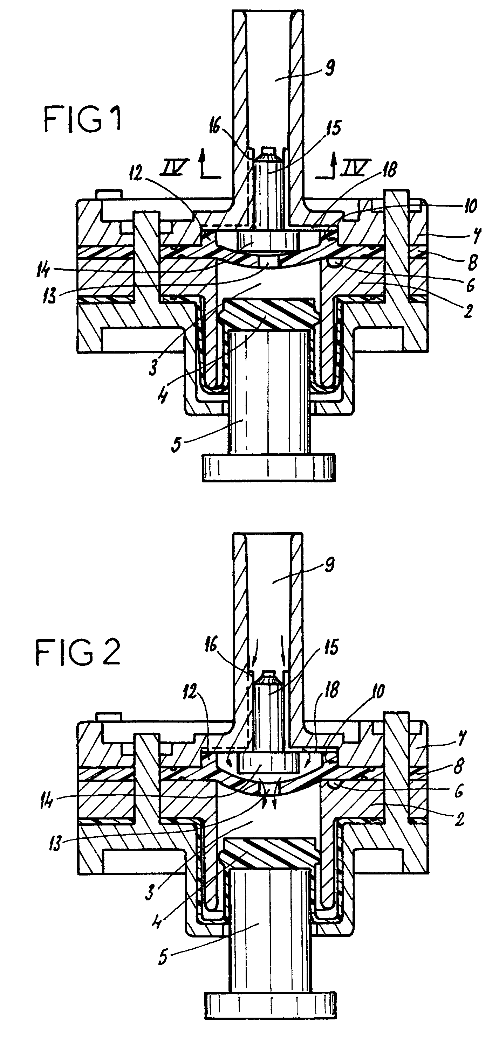

La pompe selon l'invention fonctionne de la façon suivante. Lorsque la pompe est dans un état statique, la membrane 8 est plaquée contre le disque 14 et l'orifice 13 est fermé par le disque. Lorsque le piston 4 est déplacé vers le bas, comme montré à la figure 2, il se produit une dépression décollant la partie centrale de la membrane 8 du disque 14, du liquide s'écoulant depuis le conduit 9 dans les canaux 17, 18 puis à travers l'orifice 13 dans le cylindre de dosage 3. A cet instant, la membrane est plaquée contre la première pièce 2 et ne permet pas la mise en communication entre le cylindre de dosage 3 et la lumière 6 d'évacuation du liquide.

Dans la phase suivante du cycle, lorsque le piston monte, la pression de liquide à l'intérieur de la chambre de dosage plaque la membrane contre le disque, et assure une déformation de la membrane vers le haut pour permettre le passage de liquide de la chambre de dosage vers la lumière 6 de sortie du liquide.

Comme il ressort de ce qui précède, l'invention apporte une grande amélioration à la technique existante en fournissant une pompe de perfusion, dans laquelle la conformation de la membrane assure la reprise élastique de cette dernière lors des différences de pression qu'elle subit de la part du liquide.

Les moyens de guidage du disque pourraient être différents, sans que l'on sorte pour autant du cadre de l'invention définie dans les revendications jointes.

## Revendications

1. Pompe de perfusion de liquides médicaux, comprenant :
- une première pièce (2) dans laquelle est ménagé un cylindre de dosage (3) qui, contenant un piston (4) animé d'un mouvement alternatif, débouche dans une des faces de la pièce, perpendiculairement à cette face,
- une seconde pièce (7) qui, prenant appui sur la face de la première pièce (2) dans laquelle débouche le cylindre (3), comporte, en regard de celui-ci, une cavité (10) de diamètre supérieur à celui du cylindre, et adaptée à communiquer avec un récipient d'alimentation en liquide,
- une membrane (8) étanche et déformable élastiquement serrée entre les deux pièces (2, 7), et comportant un orifice (13) en regard du cylindre (3),
- un disque (14) logé dans la cavité (10) de la seconde pièce (7), dont la face tournée vers la première pièce (2) est adaptée à prendre appui sur la membrane (8),
- associée au cylindre (3), une lumière (6) débouchant dans la même face de la première pièce (2) que le cylindre (3), cette lumière (6) associée à une tubulure d'évacuation ménagée dans cette pièce, étant disposée à proximité du cylindre (3), en regard de la cavité (10) de la seconde pièce, mais au-delà du cylindre de dosage (3), et étant recouverte par la membrane (8) serrée entre les deux pièces,
**caractérisée en ce que** la membrane (8) comporte, sur sa face située du côté de l'amenée du liquide et dans la zone de la cavité (10) ménagée dans la seconde pièce à l'extérieur du cylindre de dosage (3), une couronne élastique (12) moulée avec la membrane et prenant appui dans le fond de la cavité (10) ménagée dans la seconde pièce, le disque (14) prenant appui sur la membrane (8) étant d'un diamètre inférieur à celui de cette cavité (10) et comportant des moyens de guidage central.

2. Pompe selon la revendication 1, **caractérisée en ce que** les moyens de guidage central du disque (14) sont constitués par une tige (15) perpendiculaire au disque et guidée en translation dans un conduit (9) d'amenée du liquide.

3. Pompe selon la revendication 2, **caractérisée en ce que** la tige (15) de guidage du disque (14) est de diamètre inférieur au diamètre du conduit (9) d'amenée du liquide, et ce conduit (9) comporte, au moins dans sa zone débouchant dans la pompe, des nervures axiales (16) faisant saillie de sa paroi intérieure, délimitant, d'une part, une zone centrale de guidage de la tige (15) du disque et, d'autre part, des canaux (17) pour le passage du liquide.

4. Pompe selon la revendication 3, **caractérisée en ce que** les canaux (17) ménagés dans la paroi du conduit (9) d'amenée du liquide sont prolongés par des canaux radiaux (18) ménagés dans le fond de la cavité (10) de la seconde pièce et débouchant au-delà de la périphérie du disque (14).

## Claims

1. Pump for perfusion of medical liquids, comprising:
- a first part (2) in which there is provided a dosage cylinder (3) which, containing a piston (4) which is driven with reciprocating motion, opens into one of the surfaces of the part, perpendicularly to this surface;
- a second part (7) which, being supported on the surface of the first part (2) into which the cylinder (3) opens, comprises opposite the latter a cavity (10) with a diameter larger than that of the cylinder, and is designed to communicate with a container for supply of liquid;
- a resiliently deformable and sealed membrane (8) which is clamped between the two parts (2, 7), and comprises an aperture (13) opposite the cylinder (3);
- a disk (14) which is accommodated in the cavity (10) in the second part (7), the surface of which that faces the first part (2) being designed to be supported on the membrane (8); and
- associated with the cylinder (3), an aperture (6) which opens into the same surface of the first part (2) as the cylinder (3), this aperture (6) which is associated with a discharge tube provided in this part being disposed in the vicinity of the cylinder (3), opposite the cavity (10) in the second part, but beyond the dosage cylinder (3), and being covered by the membrane (8) clamped between the two parts,
**characterised in that**, on its surface which is situated on the side on which the liquid is introduced and in the area of the cavity (10) provided in the second part on the exterior of the dosage cylinder (3), the membrane (8) comprises a resilient ring (12) which is moulded together with the membrane and is supported in the base of the cavity (10) provided in the second part, the disc (14) which is supported on the membrane (8) having a diameter which is smaller than that of this cavity (10) and comprising central guiding means.

2. Pump according to claim 1, **characterised in that** the central guiding means for the disc (14) consist of a rod (15) which is perpendicular to the disc and is guided in translation in a duct (9) for introduction of the liquid.

3. Pump according to claim 2, **characterised in that** the rod (15) for guiding the disc (14) has a diameter smaller than the diameter of the duct (9) for introduction of the liquid, and this duct (9) comprises, at least in its area which opens into the pump, axial ribs (16) which project from its inner wall, and delimit firstly a central area for guiding of the rod (15) of the disc, and secondly channels (17) for passage of the liquid.

4. Pump according to claim 3, **characterised in that** the channels (17) provided in the wall of the duct (9) for introduction of the liquid are extended by radial channels (18) which are provided in the base of the cavity (10) in the second part and open beyond the periphery of the disk (14).

## Patentansprüche

1. Perfusionspumpe für medizinische Flüssigkeiten umfassend;
- ein erstes Teil (2), in dem ein Dosierzylinder (3) angeordnet ist, der in eine der Flächen des Teils senkrecht zu dieser Fläche mündet, wobei der Dosierzylinder (3) einen hin- und herbewegbaren Kolben (4) umfasst,
- ein zweites Teil (7), das an der Fläche des ersten Teils (2) anliegt, in die der Zylinder (3) mündet, das diesem zugewandt einen Hohlraum (10) mit größerem Durchmesser als dem des Zylinders umfasst und angepasst ist, mit einem Flüssigkeitsversorgungsbehälter zu kommunizieren,
- eine dichte und elastisch verformbare Membran (8), die zwischen den zwei Teilen (2,7) eingeklemmt ist und eine dem Zylinder (3) zugewandte Öffnung (13) umfasst,
- eine in dem Hohlraum (10) des zweiten Teils (7) aufgenommene Scheibe (14), deren dem ersten Teil (2) zugewandte Fläche angepasst ist zur Anlage an der Membran (8),
- wobei dem Zylinder (3) eine Öffnung (6) zugeordnet ist, die in die gleiche Fläche des ersten Teils (2) wie der Zylinder (3) mündet, wobei diese Öffnung (6) einer in diesem Teil angeordneten Entleerungsleitung zugeordnet ist, und in der Nähe des Zylinders (3) dem Hohlraum (10) des zweiten Teils zugewandt, aber jenseits des Dosierzylinders (3) angeordnet ist, und wobei diese Öffnung (6) durch die zwischen den zwei Teilen eingeklemmte Membran (8) bedeckt ist,
**dadurch gekennzeichnet, dass**
die Membran (8) auf ihrer Fläche, die auf der Seite der Flüssigkeitszufuhr gelegen ist, und in dem Bereich des in dem zweiten Teil außerhalb des Dosierzylinders (3) angeordneten Hohlraums (10) einen an die Membran angeformten elastischen Ringansatz (12) umfasst, der am Boden des in dem zweiten Teil angeordneten Hohlraums (10) anliegt, wobei die Scheibe (14), die an der Membran (8) anliegt, einen kleineren Durchmesser aufweist als dieser Hohlraum (10) und Zentralführungsmittel umfasst.

2. Pumpe nach Anspruch 1, **dadurch gekennzeichnet, dass** die Zentralführungsmittel der Scheibe (14) von einem zur Scheibe (14) senkrechten Schaft (15), der in einer Ftüssigkeitszuführungsleitung (9) verlagerbar geführt ist, gebildet sind.

3. Pumpe nach Anspruch 2, **dadurch gekennzeichnet, dass** der Schaft (15) zur Führung der Scheibe (14) einen kleineren Durchmesser als die Flüssigkeitszuführungsleitung (9) aufweist und diese Leitung (9) wenigstens in ihrem in die Pumpe einmündendten Bereich axiale Rippen (16) umfasst, die von ihrer inneren Wand abstehen, und einerseits einen zentralen Führungsbereich für den Schaft (15) der Scheibe und andererseits Kanäle (17) für den Flüssigkeitsdurchgang begrenzen.

4. Pumpe nach Anspruch 3, **dadurch gekennzeichnet, dass** die in der Wand der Flüssigkeitszuführungsleitung (9) angeordneten Kanäle (17) durch radiale Kanäle (18) verlängert sind, die im Boden des Hohlraums (10) des zweiten Teils angeordnet sind und jenseits des Umfangs der Scheibe (14) münden.
